(19) Europäisches Patentamt
European Patent Office
Office européen des brevets

(11) **EP 3 145 394 B1**

(12) **EUROPÄISCHE PATENTSCHRIFT**

(45) Veröffentlichungstag und Bekanntmachung des Hinweises auf die Patenterteilung:
**27.02.2019 Patentblatt 2019/09**

(51) Int Cl.:
***A61B 5/021*** *(2006.01)*     ***A61B 5/00*** *(2006.01)*

(21) Anmeldenummer: **15724629.9**

(22) Anmeldetag: **22.05.2015**

(86) Internationale Anmeldenummer:
**PCT/EP2015/061371**

(87) Internationale Veröffentlichungsnummer:
**WO 2015/177336 (26.11.2015 Gazette 2015/47)**

(54) **VERFAHREN UND VORRICHTUNG ZUR BESTIMMUNG DES ZENTRALEN SYSTOLISCHEN BLUTDRUCKS**

METHOD AND DEVICE FOR DETERMINING CENTRAL SYSTOLIC BLOOD PRESSURE

PROCÉDÉ ET DISPOSITIF DE DÉTERMINATION DE LA PRESSION SANGUINE SYSTOLIQUE CENTRALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2014 DE 102014007519**

(43) Veröffentlichungstag der Anmeldung:
**29.03.2017 Patentblatt 2017/13**

(73) Patentinhaber: **Fresenius Medical Care Deutschland GmbH**
**61352 Bad Homburg (DE)**

(72) Erfinder: **ZHANG, Wei**
**97464 Niederwerrn (DE)**

(74) Vertreter: **Breuninger, Marcus**
**Fresenius Medical Care**
**AG & Co. KGaA**
**Global Patents & IP**
**Else-Kröner-Strasse 1**
**61352 Bad Homburg (DE)**

(56) Entgegenhaltungen:
**US-A1- 2012 095 351**

- **BRYAN WILLIAMS ET AL: "Development and Validation of a Novel Method to Derive Central Aortic Systolic Pressure From the Radial Pressure Waveform Using an N-Point Moving Average Method", JOURNAL OF THE AMERICAN COLLEGE OF CARDIOLOGY, ELSEVIER, NEW YORK, NY, US, Bd. 57, Nr. 8, 17. September 2010 (2010-09-17), Seiten 951-961, XP028221499, ISSN: 0735-1097, DOI: 10.1016/J.JACC.2010.09.054 [gefunden am 2010-12-28]**
- **Y.-T. SHIH ET AL: "Application of the N-Point Moving Average Method for Brachial Pressure Waveform-Derived Estimation of Central Aortic Systolic Pressure", HYPERTENSION, Bd. 63, Nr. 4, 1. April 2014 (2014-04-01), Seiten 865-870, XP55202082, ISSN: 0194-911X, DOI: 10.1161/HYPERTENSIONAHA.113.02229**

## Beschreibung

## Technisches Gebiet

Die Erfindung betrifft eine Vorrichtung zur Bestimmung des zentralen systolischen Blutdrucks und ein Verfahren zur Bestimmung des zentralen systolischen Blutdrucks.

## Stand der Technik

Bei Dialysepatienten sind kardiovaskuläre Erkrankungen die Haupttodesursache. Dabei spielen insbesondere eine starke Gefäßkalzifikation und damit einhergehend eine Versteifung der zentralen Blutgefäße (insbesondere der Aorta) eine große Rolle. Aus diesem Grund besteht großes Interesse an der Überwachung der zentralen Gefäßsteifigkeit bei Dialysepatienten.

Da sich eine Pulswelle in einem steiferen Blutgefäß schneller bewegt, kann die Pulswellengeschwindigkeit (PWV) als Indikator für die Gefäßsteifigkeit gemessen und ausgewertet werden.

Des Weiteren ist zur Erkennung kardiovaskulärer Erkrankungen der Wert des zentralen Blutdrucks eine weitere wichtige Kennzahl. Der herkömmlich beispielsweise durch eine Manschette am Oberarm gemessene periphere Blutdruck lässt kaum Rückschlüsse auf die Höhe des zentralen Blutdrucks zu, unter anderem da die Kalzifikation der zentralen Gefäße deutlich schneller voranschreitet als die der peripheren. Der tatsächliche zentrale Blutdruck lässt sich eindeutig dadurch bestimmen, dass ein Katheder in die steigende Aorta eingeführt wird und mittels des Katheders der Aorta-Blutdruck gemessen wird. Derartige invasive Messungen sind zwar "goldener Standard", können jedoch im Patienteninteresse keinesfalls routinemäßig durchgeführt werden, um die Entwicklung des zentralen Blutdrucks langfristig zu beobachten.

Es wurde daher von Karamanoglu et al. ("An analysis of the relationship between central aortic and peripheral upper limb pressure waves in man." European Heart Journal 14, 160-167, 1993) vorgeschlagen, eine brachiale Druckpulskurve, d.h. eine Messkurve des Blutdrucks, der am Oberarm gemessen wird, über eine so genannte generalisierte Übertragungsfunktion (GTF = "generalized transfer function") in eine zentrale Pulskurve zu transformieren. Die generalisierte Übertragungsfunktion wird durch Messungen an einem Probandenstamm bestimmt, bei denen invasiv der tatsächliche Aortenblutdruck bestimmt wird, und rechnerisch eine Funktion bestimmt wird, die die brachial gemessene Druckpulskurve in die gemessene zentrale Druckpulskurve überführt. Nachteilig hierbei ist, dass die Übertragung ein Umrechnen des zeitlich aufgelösten Messsignals in die Frequenzdomäne erfordert, das Transformieren mittels der Übertragungsfunktion und ein Rücktransformieren des so erhaltenen Signals in die Zeitdomäne. Diese mehrfache Signalbearbeitung ist aufwändig und erfordert entsprechende Rechenkapazitäten, die im Rahmen einer Dialysebehandlung nicht oder nur unter großem Aufwand zur Verfügung gestellt werden können.

Ein weiteres Verfahren zur Bestimmung des zentralen Aortadrucks aus dem brachialen Blutdruck ist bekannt von Y.-T. SHIH ET AL: "Application of the N-Point Moving Average Method for Brachial Pressure Waveform-Derived Estimation of Central Aortic Systolic Pressure",HYPERTENSION, Bd. 63, Nr. 4, 1. April 2014 (2014-04-01), Seiten 865-870.

## Darstellung der Erfindung

Aufgabe der vorliegenden Erfindung ist, die genannten Nachteile des Stands der Technik zu überwinden und ein Verfahren vorzuschlagen, mit dem mit geringem apparativem und rechnerischem Aufwand der zentrale systolische Blutdruck abgeschätzt werden kann. Aufgabe ist weiterhin, eine Vorrichtung anzugeben, mit der der zentrale systolische Blutdruck abgeschätzt werden kann, sowie eine Blutbehandlungsvorrichtung, die eine derartige Vorrichtung enthält.

Die Aufgabe wird mit einem Verfahren gemäß Anspruch 1 gelöst.

Ein gleitender Mittelwertfilter kann ein Tiefpass sein, bei dem gefilterte Signalwerte jeweils durch die Mittelung einer Gruppe von zeitlich vor und nach dem jeweiligen Messpunkt liegenden Signalwerten - also bei der vorliegenden Erfindung der ermittelten peripheren Druckwerte aufgetragen über die Zeit - gebildet werden. Dieses Zeitfenster einer Gruppe von Signalwerten des peripheren Blutdruckverlaufs wird für die Berechnung des gefilterten Blutdruckverlaufs sukzessive auf der Zeit-Achse bewegt.

Das erfindungsgemäße Verfahren hat den Vorteil, dass mit sehr einfachen Mitteln nicht-invasiv der zentrale systolische Blutdruck bestimmt werden kann. Dazu müssen keine aufwändigen Rechenverfahren durchgeführt werden, wie Fourier-Transformationen, so dass nur eine geringe Rechenkapazität und -zeit nötig ist. Auch muss zur Mittelwertbildung stets nur die für den jeweiligen gemittelten Signalwert nötige Gruppe an Signalwerten zwischengespeichert werden. Sobald daraus der Mittelwert bestimmt ist, kann die Gruppe an Signalwerten durch die darauffolgende Gruppe überschrieben werden. Dies ermöglicht die Durchführung der Berechnung beispielsweise in handelsüblichen Mikrocontrollern. Wie tatsächlich die Rechenkapazität genutzt wird, kann vom Fachmann entschieden werden, so dass die genannte Durchführung einer Überschreibung nur beispielhaft ist und nicht beschränkend auf die Offenbarung der Erfindung zu sehen ist.

Nach einem Aspekt des Verfahrens kann der periphere Blutdruckverlauf zunächst als relativer Verlauf bestimmt werden und ein systolischer und ein diastolischer Druck des Patienten absolut bestimmt werden. Ein absoluter peripherer Blutdruckverlauf kann durch Kalibrierung des ermittelten relativen Blutdruckverlaufs mit dem systolischen und diastolischen Druck bestimmt werden.

Der relative periphere Blutdruckverlauf, der systolische Druck, der diastolische Druck und/oder der Puls des Patienten können oszillometrisch gemessen werden. Dies ermöglicht, den Blutdruckverlauf und die genannten Werte mit einer herkömmlichen Manschette zur Bestimmung des brachialen Blutdrucks durch Anlegen am Oberarm zu bestimmen. Diese Bestimmung kann beispielsweise erfolgen, wenn bei dem Patient eine herkömmliche Blutdruckmessung vorgenommen wird. Dies kann im Rahmen einer regelmäßigen Behandlung, wie etwa einer extrakorporalen Blutbehandlung, erfolgen. Dies hat den Vorteil, dass eine regelmäßige Überwachung des zentralen systolischen Blutdrucks möglich ist, da die Messung mit sehr geringem Aufwand erfolgen kann.

Der Puls kann als Herzrate HR (in bpm, d.h. Schläge pro Minute = "beats per minute") oder als Herzfrequenz $f_{HR}$ (in Hz) angegeben werden.

Die Manschette kann des Weiteren allgemein dafür genutzt werden, um eine Blutstauung in einer Vene zu erzielen. Dadurch kann eine Punktierung in die Vene, beispielsweise für eine Infusion, erleichtert werden. Es kann vorgesehen sein, die Manschette auf einen vorbestimmten Druck aufzupumpen. Der Vorgang des Aufpumpens kann beispielsweise mittels Betätigungseinrichtung, wie etwa einer Taste, erfolgen.

Erfindungsgemäss wird der gleitende Mittelwertfilter derart auf den Blutdruckverlauf angelegt, dass ein Bruchteil des Betrags einer Abtastfrequenz zur Messung des peripheren Blutdruckverlaufs, mit der die Signalwerte aufgenommen werden, als Fensterbreite eines Zeitfensters einer Gruppe von Messpunkten des peripheren Blutdruckverlaufs gewählt wird. Ein Nenner zur Bildung des Bruchteils kann zwischen 3 und 5 liegen. Insbesondere kann er zwischen 3,8 und 4,2 liegen und beispielsweise etwa 4 oder genau 4 betragen. In dem Zahlbereich ergeben sich im Vergleich mit tatsächlich, invasiv gemessenen Werten besonders exakte Ergebnisse des zentralen systolischen Blutdrucks. Bei einem Puls des Patienten von 70 bpm liefern Werte des Nenners zwischen 3, 8 und 4,2, wie etwa 4 oder genau 4, besonders exakte Werte für den zentralen systolischen Blutdruck. Die Abtastfrequenz, deren Betrag durch den Nenner n geteilt wird, ist in Hertz (Hz) angegeben. Es kann vorteilhaft sein, wenn der Nenner n ganzzahlig ist, dies ist jedoch keine Voraussetzung bei dieser Variante des Verfahrens. Die sich ergebenden Brüche aus Abtastfrequenz und Nenner n werden vorteilhaft auf ganze Zahlen gerundet.

Nach einer Variante des Verfahrens wird das Zeitfenster mit einer Fensterbreite von 2m + 1 auf der Zeitachse um einen jeweiligen Messpunkt des peripheren Blutdruckverlaufs gelegt, wobei m eine ganzzahlige Variable zur Festlegung einer Anzahl von Messpunkten ist. Dabei ist das Fenster vorteilhaft symmetrisch auf den jeweiligen Messpunkt gelegt, so dass links und rechts des Messpunkts jeweils m benachbarte Messpunkte liegen, die zusammen mit dem Messpunkt selbst die Gruppe an Messpunkten bilden, von denen der jeweilige Mittelwert gebildet wird.

Der Allpassfilter kann dadurch angelegt werden, dass ein Offset auf den mittelwertgefilterten Blutdruckverlauf addiert wird.

Vorteilhaft wird der gefilterte Blutdruckverlauf y(k) nach einer Filtergleichung (1) bestimmt:

$$y(k) = A \cdot x(k) + B \cdot \frac{1}{2m+1} \cdot \sum_{i=-m}^{m} x(k+i) \qquad (1)$$

Das Offset lässt sich durch einen Allpasskoeffizienten A in Verbindung mit dem absoluten peripheren Blutdruckverlauf x(k) ausdrücken. B ist ein Mittelwertfilterkoeffizient, wobei A + B = 1. k ist der Index der Messpunkte des peripheren Blutdruckverlaufs und i der Index benachbarter Messpunkte zur Mittelwertbestimmung. Mit Gleichung (1) lässt sich sehr einfach ein gefilterter Blutdruckverlauf y(k) berechnen, der geeignet ist, den zentralen systolischen Blutdruck als dessen Maximum zu bestimmen.

Besonders bevorzugt werden der Allpasskoeffizient A, der Mittelwertfilterkoeffizient B und die Fensterbreite N dadurch ermittelt, dass eine der Filtergleichung entsprechende Übertragungsfunktion einer Kalibrierübertragungsfunktion angenähert wird.

Dies ermöglicht eine Approximation an eine Kalibrierübertragungsfunktion zur Übertragung eines peripheren Blutdruckverlaufs in einen zentralen systolischen Blutdruckverlauf, von dem vorteilhaft bekannt ist, dass er den tatsächlichen systolischen Blutdruckverlauf der Aorta besonders exakt wiedergibt.

A und B können beispielsweise zwischen 0,2 und 0,8 liegen, insbesondere zwischen 0,3 und 0,7, insbesondere zwischen 0,4 und 0,6. Sie können beispielsweise beide einen Wert von 0,5 besitzen.

Die Übertragungsfunktion von Gleichung (1) lässt sich beispielsweise mit Gleichung (2) ausdrücken:

$$H(f) = A + \frac{B}{N} \cdot \frac{\sin(\pi N f / f_s)}{\sin(\pi f / f_s)} \tag{2}$$

Dabei ist f die Frequenz, $f_S$ die Abtastfrequenz und N (= 2m + 1) die Fensterbreite. Die Übertragungsfunktion ist in der Frequenzdomäne dargestellt statt in der Zeitdomäne wie der gefilterte Blutdruckverlauf.

Nach einer ersten Variante kann die Kalibrierübertragungsfunktion eine generalisierte Übertragungsfunktion (GTF) sein. Eine solche generalisierte Übertragungsfunktion kann aus Messungen an einem Stamm an Patienten und/oder Probanden bestimmt werden, die nicht notwendigerweise mit dem Patienten selbst übereinstimmen. Aus dem Patienten-/ Probandenstamm kann ein Verlauf des zentralen systolischen Blutdrucks invasiv gemessen werden. Aus den einzelnen zentralen Blutdruckkurven des Patienten-/ Probandenstamms lässt sich eine gemittelte Gesamtkurve als eine Kalibrierblutdruckkurve erstellen. Diese lässt sich mit einer peripheren systolischen Blutdruckkurve desselben Patienten-/ Probandenstamms vergleichen und mittels einer zu bestimmenden generalisierten Übertragungsfunktion ineinander überführen. Dies hat den Vorteil, dass kein invasives Verfahren am Patienten vorgenommen werden muss. Das dafür notwendige Legen eines Herzkatheders kann somit vermieden werden. Die generalisierte Übertragungsfunktion muss zwar eventuell mit relativ hohem rechnerischem Aufwand ermittelt werden, steht jedoch dann als Kalibrierübertragungsfunktion für das erfindungsgemäße Verfahren zur Verfügung, ohne für die Anwendung des Verfahrens gemäß der Erfindung jedes Mal neu berechnet werden zu müssen.

Des Weiteren besteht die Möglichkeit, auf generalisierte Übertragungsfunktionen als Kalibrierübertragungsfunktion zurückzugreifen, die bereits vorliegen, da sie beispielsweise aus der Literatur bekannt sind. Dies verringert den Aufwand zur Gewinnung einer Kalibrierübertragungsfunktion.

Nach einer weiteren Variante kann die Kalibrierübertragungsfunktion von einer zentralen systolischen Blutdruckkurve als einer Kalibrierblutdruckkurve bestimmt werden, die invasiv, insbesondere an der steigenden Aorta und/oder der Aortenwurzel des Patienten selbst gemessen ist. Dies stellt sicher, dass der tatsächliche zentrale systolische Blutdruckverlauf und damit auch der zentrale systolische Blutdruck als dessen Maximum bekannt sind. Diese Kalibrierdruckkurve erfüllt per se die Eigenschaft, dass Einflüsse, wie etwa Alter, Krankheitsbild oder Medikamentierung berücksichtigt sind. Dies ist insbesondere vorteilhaft, wenn am Patient ohnehin eine Kathederuntersuchung vorgenommen wird, so dass kein zusätzlicher Eingriff nötig ist. Bei dieser Variante kann der einzelne Patient als Spezialfall eines Patientenstamms betrachtet werden.

Es kann bevorzugt sein, die Fensterbreite mit einem pulsabhängigen (d.h. herzraten- bzw. herzfrequenzabhängigen) Korrekturfaktor zu einer korrigierten Fensterbreite zu korrigieren. Dadurch können auch bei Pulswerten, die von einer Herzrate im üblichen Bereich nach oben oder unten abweichen, besonders gute Werte für den zentralen systolischen Blutdruck erreicht werden. Insbesondere bei Herzraten, die kleiner als 50 bpm oder höher als 90 bpm betragen, ist eine Korrektur mit einem pulsabhängigen Korrekturfaktor vorteilhaft. Aber auch schon bei weniger als 60 bpm oder mehr als 80 bpm kann eine pulsabhängige Korrektur vorteilhaft sein. Jedoch kann es auch vorteilhaft sein, in der Nähe zu einem Puls von 70 bpm eine Korrektur vorzunehmen, etwa bei 65 oder 75 pbm.

Nach einer Variante wird die pulskorrigierte Fensterbreite als Betrag eines Quotienten aus Abtastfrequenz und pulskorrigiertem Nenner berechnet. Dabei wird der pulskorrigierte Nenner selbst berechnet durch Multiplikation des Nenners aus der Bestimmung der Fensterbreite für den Patientenstamm, der eine Referenzherzrate $HR_{Ref}$ besitzt, mit einem Quotienten aus der aktuellen Herzrate des Patienten und der Referenzherzrate $HR_{Ref}$. Unter der aktuellen Herzrate des Patienten wird insbesondere die Herzrate verstanden, die bei der Bestimmung des peripheren, insbesondere brachialen, zeitlichen Blutdruckverlaufs vorliegt. Die Referenzherzrate ist eine selbe Herzrate $HR_{Ref}$ wie bei der Ermittlung der Kalibrierdruckkurve. Als Herzrate eines Patientenstamms wird hier eine berechnete mittlere Herzrate bezeichnet, die der Patientenstamm bei der Messung der zentralen und der peripheren Blutdruckkurve hatte. Dies lässt sich durch Gleichung (3) ausdrücken, wobei $f_S$ die Abtastfrequenz in Hertz ist, $HR_{Pat}$ die aktuelle Herzrate, $HR_{Ref}$ die Referenzherzrate, n der Nenner und k ein Korrekturfaktor, der beispielsweise 1 beträgt, aber je nach notwendigen Korrekturen zwischen 0,5 und 1,5 liegen kann.

$$N = \left| \frac{f_s}{nk} \cdot \frac{1}{HR_{Pat}/HR_{Ref}} \right| \tag{3}$$

Nach einem weiteren Aspekt der Erfindung kann der gefilterte Blutdruckverlauf eine Phasenverschiebung des Mittelwertfilters enthalten, was sich in einer Gleichung (4) darstellen lässt, die Gleichung (2) um einen Phasenwinkel $\varphi$ ergänzt.

$$y(k) = A \cdot x(k) + B \cdot \frac{1}{2m+1} \cdot \sum_{i=-m}^{m} x(k + i - \varphi) \hspace{3cm} (4)$$

Somit können vorteilhaft die genaue Kurvenform des zentralen Blutdrucks und daraus weitere kardiovaskuläre Kennwerte bestimmt werden. Das Maximum des erfindungsgemäß bestimmten zentralen systolischen Blutdruckverlaufs als dem gefilterten Blutdruckverlauf ist vorteilhaft mit und ohne Berücksichtigung des Phasenwinkels gleich.

Die zu Gleichung (4) korrespondierende Übertragungsfunktion lässt sich bei einer Variante der Erfindung unter Einbeziehung des Phasenwinkels φ durch Gleichung (5) ausdrücken. Somit lassen sich der Allpasskoeffizient A, der Mittelwertfilterkoeffizient B, die Fensterbreite N und/oder der Nenner n unter Einbeziehung des Phasenwinkels φ ermitteln.

$$H(f) = A + \frac{B}{N} \cdot \frac{\sin(\pi N f / f_s)}{\sin(\pi f / f_s)} * e^{-j2\pi\varphi f / f_s} \hspace{3cm} (5)$$

Die Aufgabe der Erfindung wird des Weiteren mit einer Vorrichtung nach Anspruch 10 gelöst.

Mit der erfindungsgemäßen Vorrichtung lässt sich durch einfache technische Mittel der zentrale systolische Blutdruck eines Patienten bestimmen. Zum Anlegen eines gleitenden Mittelwertfilters und eines Allpassfilters sind nur eine geringe Rechenleistung und eine geringe Speicherkapazität nötig. Die Blutdruckmess-einrichtung kann eine herkömmliche Blutdruckmesseinrichtung zur Messung eines peripheren Blutdrucks, insbesondere eines brachialen Blutdrucks sein. Die Blutdruckmesseinrichtung kann den peripheren Blutdruckverlauf selbst aufzeichnen, oder die Auswerteinheit kann den peripheren Blutdruckverlauf bestimmen, indem sie einzelne Messwerte der Blutdruckmesseinrichtung zu einem Verlauf zusammenfügt. Dies ist für die Erfindung nicht relevant. Der Einfachheit halber werden beide Varianten mit der Formulierung zusammengefasst, dass die Blutdruckmesseinrichtung zur Bestimmung eines peripheren Blutdruckverlaufs vorgesehen ist.

Nach einem Aspekt kann die Blutdruckmesseinrichtung geeignet sein, den peripheren Blutdruckverlauf relativ zu bestimmen, und einen systolischen und diastolischen Blutdruck des Patienten absolut zu bestimmen. Nach einem weiteren Aspekt kann die Auswerteinheit konfiguriert sein, einen absoluten peripheren Blutdruckverlauf durch Kalibrierung des relativen Blutdruckverlaufs mit dem absoluten systolischen und diastolischen Blutdruck zu bestimmen. Alternativ kann auch die Blutdruckmesseinrichtung selbst über die notwendigen Einrichtungen verfügen, um einen absoluten peripheren Blutdruckverlauf durch Kalibrierung des relativen Blutdruckverlaufs mit dem absoluten systolischen und diastolischen Blutdruck zu bestimmen.

Vorteilhaft ist die Auswerteinheit konfiguriert, das erfindungsgemäße Verfahren in einer der oben beschriebenen Ausführungsformen oder Alternativen auszuführen, einer Kombination aus mehreren der beschriebenen Ausführungsformen oder Alternativen oder allen der beschriebenen Ausführungsformen oder Alternativen des erfindungsgemäßen Verfahrens auszuführen.

Vorteilhaft sind in der Auswerteinheit eine Übertragungsfunktion oder eine Kalibrierübertragungsfunktion, ein Allpasskoeffizient A, ein Mittelwertfilterkoeffizient B, eine Fensterbreite N und/oder ein Nenner n gespeichert, so dass auf diese bei der Bestimmung des zentralen systolischen Blutdrucks zurückgegriffen werden kann. Diese gespeicherten Daten können beispielsweise auf einer sogenannten Patientenkarte vorliegen, die eine Speicherkarte ist, die mit der Auswerteinheit in Verbindung steht bzw. auf die die Auswerteinheit zugreifen kann.

Es kann auch vorteilhaft sein, dass die Auswerteinheit eine Recheneinheit aufweist, die konfiguriert ist, Verfahrensschritte, die eine Berechnung einer Übertragungs-funktion oder einer Kalibrierübertragungsfunktion, eine Bestimmung des Allpasskoeffizienten A, des Mittelwertfilterkoeffizienten B, der Fensterbreite N und/oder des Nenners n umfassen, auszuführen.

Die Recheneinheit kann vorteilhaft als separater Teil der Auswerteinheit vorliegen.

Durch eine Trennung der Recheneinheit von der restlichen Auswerteinheit lässt sich vorteilhaft erreichen, dass die Auswerteinheit mit einer Rechenkapazität und einem Speichervolumen ausgestattet ist, die lediglich groß genug ausgebildet sein müssen, um die Filterung des peripheren Blutdruckverlaufs durchzuführen, aber nicht für aufwändigere Berechnungen geeignet sein müssen, die selten, beispielsweise nur einmal im Lauf einer mehrjährigen Messreihe, durchgeführt werden müssen.

Gemäss Anspruch 13 weist die Blutbehandlungsvorrichtung eine erfindungsgemäße Vorrichtung zur Bestimmung des zentralen systolischen Blutdrucks eines Patienten auf. Dies ermöglicht, dass bei Patienten, die mit einer Blutbehandlungsvorrichtung behandelt werden, bei dieser Gelegenheit ebenfalls der zentrale systolische Blutdruck bestimmt wird, beispielsweise neben einer herkömmlichen brachialen Blutdruckmessung. Im Falle einer regelmäßigen Blutbehandlung kann bei dieser Gelegenheit auch der zentrale systolische Blutdruck regelmäßig bestimmt werden. Die Blutbehandlungsvorrichtung kann beispielsweise eine Hämodialyse-, Hämofiltrations- oder Hämodiafiltrations-vorrichtung sein.

Nach einer Variante der Blutbehandlungsvorrichtung ist die Vorrichtung zur Bestimmung des zentralen systolischen Blutdrucks eines Patienten mit einer Vorrichtung zur Messung und/oder Überwachung des Blutdrucks des Patienten, an dem eine Blutbehandlung vorgenommen wird, kombiniert, wobei die Vorrichtung zur Messung und/oder Überwachung des Blutdrucks der Blutbehandlungs-vorrichtung zugeordnet ist.

Weiterhin kann die Vorrichtung zur Bestimmung des zentralen systolischen Blutdrucks eines Patienten mit einer Vorrichtung zur Bestimmung der Blutgefäßsteifigkeit kombiniert sein. Bei dieser kann die Pulswellengeschwindigkeit als Indikator für die Gefäßsteifigkeit gemessen und ausgewertet werden. Zusammen mit der Gefäßsteifigkeit lässt der Wert des zentralen systolischen Blutdrucks noch genauere Aussagen über eventuelle kardiovaskuläre Erkrankungen zu. Vorteilhaft kann die Auswerteinheit konfiguriert sein, sowohl Kennzahlen für die Gefäßsteifigkeit, wie etwa Pulswellengeschwindigkeiten, auszuwerten, als auch das erfindungsgemäße Verfahren durchzuführen.

Nach einem weiteren Aspekt kann die Vorrichtung zur Bestimmung des zentralen systolischen Blutdrucks selbst und/oder die Blutbehandlungsvorrichtung eine Speichervorrichtung besitzen, die konfiguriert ist, dass sie den erfindungsgemäß bestimmten zentralen systolischen Blutdruck und optional weitere Daten, die im Zusammenhang mit der Bestimmung des zentralen systolischen Blutdrucks gemessen oder bestimmt werden, speichern kann, wie etwa den brachialen systolischen und diastolischen Blutdruck, den mittleren Blutdruck und/oder eine Pulskurve. Die Speichervorrichtung kann derart ausgebildet sein, dass sie ein Speichermedium aufnehmen kann, auf dem die entsprechenden Daten gespeichert werden. Dabei wird vorzugsweise je Patient ein Speichermedium eingesetzt. Dies kann beispielsweise eine sogenannte Patientenkarte sein. Dadurch wird gewährleistet, dass patientenbezogene Daten auf einem dem jeweiligen Patienten zugeordneten Speichermedium verbleiben und entsprechend gesichert sind.

**Kurze Beschreibung der Zeichnungen**

Weitere Vorteile und Ausführungsformen der Erfindung werden beispielhaft anhand eines Ausführungsbeispiels unter Bezug auf die Figuren erläutert.

Dabei zeigen

Fig. 1 schematisch eine Anordnung einer erfindungsgemäßen Vorrichtung zur Bestimmung des zentralen systolischen Blutdrucks in Verbindung mit einer Blutbehandlungsvorrichtung, an die ein Patient angeschlossen ist,

Fig. 2 ein Vergleich einer mit einem erfindungsgemäßen Verfahren bestimmten Übertragungsfunktion im Vergleich zu einer GTF (generalized transfer function) als Kalibrierübertragungsfunktion, und

Fig. 3 ein Flussdiagramm eines Ausführungsbeispiels des erfindungsgemäßen Verfahrens.

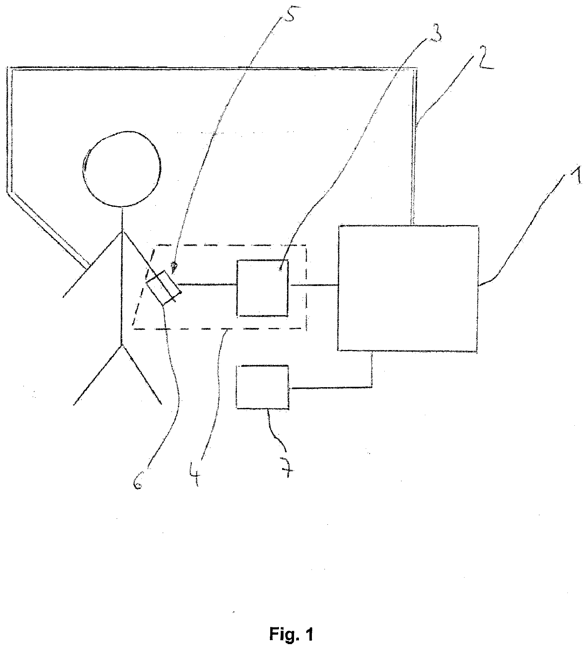

Eine erfindungsgemäße Blutbehandlungsvorrichtung 1 ist beispielsweise geeignet zur Durchführung einer Dialyse, einer Filtration und/oder einer Diafiltration, bei der Blut eines Patienten behandelt wird, das durch Blutleitungen 2 in die Blutbehandlungsvorrichtung 1 und zurück geleitet wird. Die Blutbehandlungs-vorrichtung 1 weist, wie in Fig. 1 schematisch gezeigt, eine Auswerteinheit 3 auf, die in diesem Ausführungsbeispiel geeignet ist, sowohl Daten, die in direkter Verbindung mit der Blutbehandlungsvorrichtung 1 stehen, auszuwerten und zu speichern, als auch die Daten einer erfindungsgemäßen Vorrichtung 4 zur Bestimmung des zentralen systolischen Blutdrucks des Patienten. Die erfindungsgemäße Vorrichtung 4 bestimmt mit der Auswerteinheit 3 einen zentralen systolischen Blutdruck als Maximum einer gefilterten Blutdruckkurve. Die gefilterte Blutdruckkurve wird erhalten, indem die Auswerteinheit 3 auf einen peripheren Blutdruckverlauf einen Allpassfilter und einen gleitenden Mittelwertfilter anlegt.

Dazu wird zunächst eine periphere Blutdruckkurve erhalten. Zu diesem Zweck wird als Blutdruckmessvorrichtung 5 ein Sphygmomanometer verwendet, das eine aufblasbare Manschette 6 besitzt, die dem Patienten an den Oberarm angelegt wird. Anschließend wird eine herkömmliche oszillometrische Blutdruckmessung durchgeführt und ein diastolischer Blutdruckwert, ein systolischer Blutdruckwert, ein mittlerer Blutdruckwert und der Puls erhalten. Die Auswerteinheit 3 ist ausgebildet und konfiguriert, diese Werte zu speichern. Die Abtastfrequenz $f_S$, mit der die Blutdruckmesswerte aufgenommen bzw. gespeichert werden, beträgt bei diesem Ausführungsbeispiel 500 Hz. Die Auswerteinheit 3 weist eine auch "Mikrocontroller" genannte Einheit auf, die mit lediglich geringer Speicher- und Rechenkapazität ausgestattet sein braucht. In diesem Fall handelt es sich um ein LPC2368 mit 16/32Bits.

Nach der herkömmlichen Blutdruckmessung wird die Manschette 6 auf den diastolischen Blutdruck aufgepumpt und der Manschettendruck für ca. 10 s gehalten. Während dieser Zeit erfasst die Druckmesseinrichtung 5 auch "Pulskurven" genannte Druckoszillationen.

Diese werden zuerst vom Gleichanteil und Hochfrequenzstörungen (> 18 Hz) befreit und für die weitere Verarbeitung in der Auswerteinheit 3 gespeichert.

Aus den aufgenommenen Pulskurven werden vorab ungeeignete Pulskurven aussortiert, wobei diese offensichtliche Ausreißer umfassen, sowie Pulskurven, deren Verlauf beispielsweise durch Bewegungen des Patienten verfälscht sind.

Anschließend wird von der Auswerteinheit 3 eine Mittelwertpulskurve gebildet, die eine periphere Blutdruckkurve repräsentiert, mit der die weiteren erfindungsgemäßen Verfahrensschritte durchgeführt werden.

Die Auswerteinheit 3 kalibriert die periphere Blutdruckkurve mit dem vorher gemessenen systolischen Blutdruckwert und diastolischen Blutdruckwert. Somit entsteht ein absoluter peripherer Blutdruckverlauf des Patienten, der von der Auswerteinheit 3 zwischengespeichert wird.

Die absolute periphere Blutdruckkurve x(k) wird mit der Filtergleichung (1)

$$y(k) = A \cdot x(k) + B \cdot \frac{1}{2m+1} \cdot \sum_{i=-m}^{m} x(k+i) \qquad (1)$$

gefiltert.

Die gefilterte Blutdruckkurve y(k) bestimmt sich durch den Allpassfilteranteil A x(k), wobei A ein Allpasskoeffizient ist, und den Mittelwertfilteranteil, wobei B der Mittelwertkoeffizient ist und 2m + 1 die Fensterbreite N des Zeitfensters, das zur gleitenden Mittelwertbildung auf die periphere Blutdruckkurve gelegt wird. Es hat sich gezeigt, dass die Fensterbreite mit dem Betrag von N = fs/n bei einem Wert des Nenners n von 4 die genauesten Werte für den zentralen systolischen Blutdruck liefern, wenn die Herzrate 70 bpm beträgt. Mit $f_S$ = 500 Hz ergibt sich eine Fensterbreite von 125. Mit dem bevorzugten Anlegen eines symmetrischen Zeitfensters ergibt sich für m der Wert 62, da N = 2m + 1. Somit wird im vorliegenden Ausführungsbeispiel ein Zeitfenster von 125 Messwerten, von denen jeweils der Mittelwert gebildet wird, auf der Zeitachse von x(k), dem absoluten peripheren Blutdruckverlauf des Patienten, sukzessive durchgeschoben. Die sich ergebenden Mittelwerte werden mit dem Koeffizienten des gleitenden Mittelwertfilters, auch "Mittelwertkoeffizient" genannt, multipliziert und mit dem Allpassfilteranteil versehen. Von dieser von der Auswerteinheit 3 errechneten gefilterten Blutdruckkurve y(k) wird von der Auswerteinheit das Maximum als zentraler systolischer Blutdruck bestimmt und zwischengespeichert.

Nachdem jeweils die Mittelwerte über das jeweilige Zeitfenster der Fensterbreite N bestimmt wurden, können die nicht mehr benötigten Messwerte und Zwischenrechnungen gelöscht oder überschrieben werden. Dies belastet den Mikrocontroller der Auswerteinheit 3 mit nur geringen Speichermengen.

In diesem Ausführungsbeispiel besitzt die Blutbehandlungsvorrichtung optional eine Vorrichtung zur Bestimmung der Blutgefäßsteifigkeit 7, die mit der Vorrichtung zur Bestimmung des zentralen systolischen Blutdrucks kombiniert sein kann. Die Vorrichtung zur Bestimmung der Blutgefäßsteifigkeit misst die Pulswellengeschwindigkeit durch die Pulskurvenanalyse als Indikator für die Gefäßsteifigkeit und wertet sie aus. Dies geschieht im vorliegenden Fall ebenfalls in der Auswerteinheit 3. Zusammen mit der Gefäßsteifigkeit lässt der Wert des zentralen systolischen Blutdrucks noch genauere Aussagen über eventuelle kardiovaskuläre Erkrankungen zu. Durch die Kombination der Vorrichtungen wird eine leicht zu bedienende, hoch kompakte Gesamteinheit erzielt, bei der mit geringem Aufwand während einer Blutbehandlung diese Kenndaten ermittelt und erfasst werden.

Durch Approximation der Filter-Übertragungsfunktion z.B. gemäß Gleichung (2) an eine Kalibrierübertragungsfunktion lassen sich A, B und N bestimmen.

$$H(f) = A + \frac{B}{N} \cdot \frac{\sin(\pi N f / f_s)}{\sin(\pi f / f_s)} \qquad (2)$$

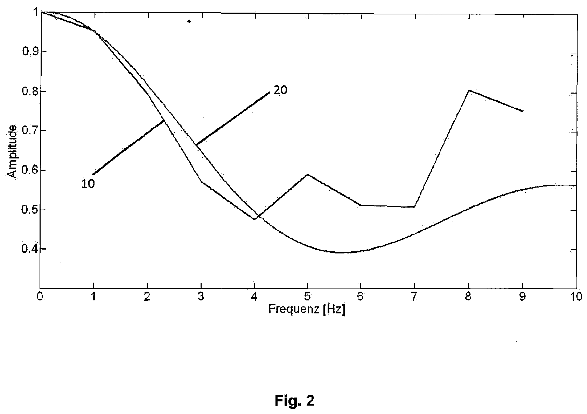

Als Kalibrierübertragungsfunktion kann beispielsweise eine bei Y.-T. Shih et al. ("Comparison of two generalized transfer functions for measuring central systolic blood pressure by an oscillometric blood pressure monitor" in: Journal of Human Hypertension 3, 204-210 (2013)) berichtete Generalisierte Übertragungsfunktion dienen, die an einem Patientenstamm bestimmt wurde. Fig. 2 zeigt den invertierten Amplitudengang dieser generalisierten Übertragungsfunktion als die mit dem Bezugszeichen 10 bezeichnete Kurve.

Man erhält z.B. bei Werten von A = B = 0,5, $F_S$ = 500 Hz, n = 4, N = 125 eine Übertragungsfunktion 20, wie sie in Fig. 2 zusätzlich zur Kalibrier-übertragungsfunktion GTF dargestellt ist. Man erkennt die sehr gute Übereinstimmung vor allem im niedrigen Frequenzbereich mit f ≤ 4 Hz.

Die beschriebene Approximation muss für den Patienten nur einmal durchgeführt werden. Daher kann sie in einer nicht dargestellten separaten Einheit der Auswerteinheit 3 stattfinden, die eine größere Rechen- und Speicherkapazität aufweist als die Auswerteinheit 3 selbst. Die Approximation kann auch an einer völlig getrennten Recheneinheit, wie etwa einem Personalcomputer, durchgeführt werden. In folgenden Sitzungen lässt sich die Vorrichtung 4 zur Bestimmung des zentralen systolischen Drucks allein mit gespeicherten Werten A, B, N, n betreiben, die in die Filtergleichung (1) eingesetzt sind. Die gespeicherten Werte können beispielsweise aufgerufen werden, indem eine Patientenkarte in die Auswerteinheit 3 eingebracht wird, die das Abrufen patientenspezifischer Daten von der separaten Einheit ermöglicht.

Im vorliegenden Ausführungsbeispiel wies der Patientenstamm, von dem die Kalibrierübertragungsfunktion stammt, bei deren Bestimmung einen durch-schnittlichen Puls von ca. 70 bpm auf, was einer Herzfrequenz von 1,17 Hz entspricht.

Der Patient des Ausführungsbeispiels besitzt bei der durchgeführten Messung der peripheren Blutdruckkurve zufälligerweise ebenfalls einen Puls von 70 pbm. Daher ist keine pulsabhängige Korrektur der beschriebenen Bestimmung des zentralen systolischen Drucks notwendig.

In einer zweiten Messung der peripheren Blutdruckkurve bei einer anderen Sitzung zur Blutbehandlung mit der erfindungsgemäßen Blutbehandlungsvorrichtung hat der Patient jedoch einen erhöhten Puls von 90 bpm und liegt somit außerhalb eines vordefinierten Kernbereichs mit einer Untergrenze $HR_L$ von hier 50 bpm und einer Obergrenze $HR_H$ von hier 85 bpm, so dass es vorteilhaft ist, gemäß Gleichung (3) eine pulskorrigierte Fensterbreite $N_1$ zu bestimmen, die aus der Fensterbreite N hervorgeht:

$$N_1 = \frac{f_S}{nk} \cdot \frac{1}{HR_{Pat}/HR_{Ref}} \tag{3}$$

Dazu wird die pulskorrigierte Fensterbreite $N_1$ des Quotienten $f_S/n_1$ berechnet, wobei $n_1$ ein pulskorrigierter Nenner ist. Der pulskorrigierte Nenner selbst berechnet sich durch Multiplikation des Nenners n aus der Bestimmung der Fensterbreite N für den Patientenstamm, der eine Referenzherzrate $HR_{Ref}$ besitzt mit einem Quotienten $HR_{Pat}/HR_{Ref}$, wobei $HR_{Pat}$ die aktuelle Herzrate des Patienten bei der Bestimmung der peripheren Blutdruckkurve ist, also im vorliegenden 90 bpm. Die Referenzherzrate $HR_{Ref}$ ist eine selbe Herzrate wie bei der Ermittlung der Kalibrierdruckkurve, also hier 70 bpm. Diese wurde als mittlere Herzrate des Patientenstamms berechnet. Somit berechnet sich $n_1$ zu 4 x 90/70 = 5,1. Die korrigierte Fensterbreite $N_1$ ergibt sich bei wiederum einer Abtastfrequenz von 500 Hz gerundet zu einer Anzahl an Messpunkten im Zeitfenster von 98.

Nach einer Variante wird der pulsabhängige Nenner $n_1$ noch mit einem Korrekturfaktor K multipliziert, der eine weitere Anpassung der gefilterten Blutdruckkurve ermöglicht, um weitere Faktoren, wie Medikation, Krankheitsbild etc. zu berücksichtigen. Im vorliegenden Fall ist K als 1 angenommen, um keine entsprechende Korrektur durchzuführen.

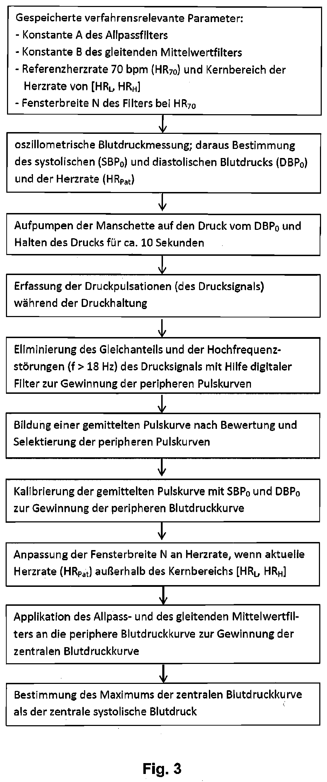

In Fig. 3 ist in einem Flussdiagramm der Ablauf des erfindungsgemäßen Verfahrens beispielhaft dargestellt, wobei hier die Werte von A, B, N bereits gespeichert vorliegen. Als Referenzherzrate $H_{Ref}$ ist in diesem Beispiel eine Herzrate von 70 bpm gewählt. Die Untergrenze $HR_L$ des Kernbereichs liegt bei 50 bpm und die Obergrenze $HR_H$ bei 85 bpm.

Alle beschriebenen Schritte können voll- oder halbautomatisch von der Blutbehandlungsvorrichtung 1 durchgeführt werden. Sie können auch ohne Blutbehandlungsvorrichtung 1 mit der Vorrichtung 4 zur Bestimmung des zentralen systolischen Drucks voll- oder halbautomatisch durchgeführt werden. Des Weiteren können sie mit einer oder beiden dieser Vorrichtung zumindest teilweise manuell bedient durchgeführt werden. Das für dieses Ausführungsbeispiel beschriebene Verfahren kann jedoch prinzipiell auch mit anderen als den beispielhaft erläuterten Messgeräten, Steuerungen bzw. Regelungen und anderen Einrichtungen ausgeführt werden.

**Patentansprüche**

1. Verfahren zur Bestimmung des zentralen systolischen Blutdrucks eines Patienten, aufweisend die Schritte

- Bestimmen eines peripheren, insbesondere brachialen, Blutdruckverlaufs
- Anlegen eines gleitenden Mittelwertfilters auf den Blutdruckverlauf
- Anlegen eines Allpassfilters auf den Blutdruckverlauf
- Bestimmen des zentralen systolischen Blutdrucks als Maximum des allpass- und mittelwertgefilterten Blutdruckverlaufs, wobei

der gleitende Mittelwertfilter derart angelegt wird, dass ein Bruchteil des Betrags einer Abtastfrequenz ($f_S$) zur Messung des peripheren Blutdruckverlaufs als eine Fensterbreite (N) eines Zeitfensters einer Gruppe von Messpunkten des peripheren Blutdruckverlaufs gewählt wird, wobei ein Nenner (n) zur Bildung des Bruchteils zwischen 3 und 6, insbesondere zwischen 3, 8 und 4,2 liegt, wobei die Fensterbreite (N) mit einem pulsabhängigen Korrekturfaktor zu einer pulskorrigierten Fensterbreite ($N_1$) korrigiert wird.

2. Verfahren nach Anspruch 1, **dadurch gekennzeichnet, dass** der periphere Blutdruckverlauf relativ bestimmt wird, ein systolischer und ein diastolischer Druck des Patienten absolut bestimmt werden, und ein absoluter peripherer Blutdruckverlauf durch Kalibrierung des relativen Blutdruckverlaufs mit dem systolischen und diastolischen Druck bestimmt wird.

3. Verfahren nach Anspruch 1 oder 2, **dadurch gekennzeichnet, dass** der relative periphere Blutdruckverlauf, der systolische Druck, der diastolische Druck und/oder der Puls des Patienten mit einem Sphygmomanometer gemessen werden.

4. Verfahren nach zumindest einem der vorhergehenden Ansprüche, **dadurch gekennzeichnet, dass** die Fensterbreite (N) mit der Breite N = 2m + 1 auf der Zeitachse um einen jeweiligen Messpunkt des gemessenen peripheren Blutdruckverlaufs gelegt wird, wobei m eine ganzzahlige Variable zur Festlegung einer Anzahl von Messpunkten ist.

5. Verfahren nach zumindest einem der vorhergehenden Ansprüche, **dadurch gekennzeichnet, dass** der Allpassfilter dadurch angelegt wird, dass ein Offset auf den mittelwertgefilterten Blutdruckverlauf addiert wird.

6. Verfahren nach Anspruch 4 oder 5, **dadurch gekennzeichnet, dass** der gefilterte Blutdruckverlauf (y(k)) nach einer ersten Filtergleichung (Gl. 1)

$$y(k) = A \cdot x(k) + B \cdot \frac{1}{2m+1} \cdot \sum_{i=-m}^{m} x(k+i)$$

oder nach einer zweiten Filtergleichung (Gl. 4)

$$y(k) = A \cdot x(k) + B \cdot \frac{1}{2m+1} \cdot \sum_{i=-m}^{m} x(k+i-\varphi)$$

bestimmt wird, mit A einem Allpasskoeffizienten und B einem Mittelwertfilterkoeffizienten, wobei A + B = 1, x(k) dem absoluten peripheren Blutdruckverlauf, k dem Index der Messpunkte des peripheren Blutdruckverlaufs und i dem Index benachbarter Messpunkte zur Mittelwertbestimmung, sowie $\varphi$ einem Phasenwinkel.

7. Verfahren nach Anspruch 6, **dadurch gekennzeichnet, dass** der Allpasskoeffizient (A), der Mittelwertfilterkoeffizient (B), die Fensterbreite (N) und/oder der Nenner (n) dadurch ermittelt werden, dass eine der Filtergleichung (Gl. 1) entsprechende Übertragungsfunktion (H(f)) einer Kalibrierübertragungsfunktion angenähert wird.

8. Verfahren nach Anspruch 7, **dadurch gekennzeichnet, dass** als Kalibrierübertragungsfunktion eine von einem Patientenstamm bestimmte generalisierte Übertragungsfunktion verwendet wird.

9. Verfahren nach zumindest einem der vorhergehenden Ansprüche, **dadurch gekennzeichnet, dass** die pulskorrigierte Fensterbreite (N₁) berechnet wird durch eine Korrekturgleichung (Gl. 3), gemäß der gilt:

$$N = \frac{|f_s|}{nk} \cdot \frac{1}{HR_{Pat}/HR_{Ref}}$$

mit $f_S$ der Abtastfrequenz in Hertz, n dem Nenner, k einem Korrekturfaktor, $HR_{Pat}$ der aktuellen Herzrate des Patienten und $HR_{Ref}$ der Referenzherzrate.

10. Vorrichtung zur Bestimmung des zentralen systolischen Blutdrucks eines Patienten, aufweisend eine Blutdruckmesseinrichtung zur Bestimmung eines peripheren, insbesondere brachialen, Blutdruckverlaufs und eine Auswerteinheit, die konfiguriert ist zum

    - Anlegen eines gleitenden Mittelwertfilters auf den Blutdruckverlauf
    - Anlegen eines Allpassfilters auf den Blutdruckverlauf
    - Bestimmen des zentralen systolischen Blutdrucks als Maximum des gefilterten Blutdruckverlaufs, wobei

der gleitende Mittelwertfilter derart angelegt wird, dass ein Bruchteil des Betrags einer Abtastfrequenz (f_S) zur Messung des peripheren Blutdruckverlaufs als eine Fensterbreite (N) eines Zeitfensters einer Gruppe von Messpunkten des peripheren Blutdruckverlaufs gewählt wird, wobei ein Nenner (n) zur Bildung des Bruchteils zwischen 3 und 6, insbesondere zwischen 3, 8 und 4,2 liegt, wobei

die Fensterbreite (N) mit einem pulsabhängigen Korrekturfaktor zu einer pulskorrigierten Fensterbreite (N$_1$) korrigiert wird.

11. Vorrichtung nach Anspruch 10, **dadurch gekennzeichnet, dass** die Blutdruckmesseinrichtung geeignet ist, den peripheren Blutdruckverlauf relativ zu bestimmen und einen systolischen und einen diastolischen Druck des Patienten absolut zu bestimmen, und die Auswerteinheit konfiguriert ist, einen absoluten peripheren Blutdruckverlauf durch Kalibrierung des relativen Blutdruckverlaufs mit dem absoluten systolischen und diastolischen Druck zu bestimmen.

12. Vorrichtung nach Anspruch 10 oder 11, **dadurch gekennzeichnet, dass** die Auswerteinheit konfiguriert ist, ein Verfahren nach zumindest einem der Ansprüche 1 bis 9 durchzuführen.

13. Blutbehandlungsvorrichtung mit einem extrakorporalen Blutkreislauf, zur Behandlung des Bluts eines Patienten, aufweisend eine Vorrichtung zur Bestimmung des zentralen systolischen Blutdrucks des Patienten nach zumindest einem der Ansprüche 10 bis 12.

**Claims**

1. A method for determining a patient's central systolic blood pressure, comprising the steps:

   - determining a peripheral, in particular brachial, blood pressure curve,
   - applying a moving-average filter to the blood pressure curve,
   - applying an all-pass filter to the blood pressure curve,
   - determining the central systolic blood pressure as the maximum of the all-pass and moving-average-filtered blood pressure curve, wherein

   the moving-average filter is applied in such a way that a fraction of the value of the scanning frequency (f$_S$) for measuring the peripheral blood pressure curve is selected as the window width (N) of a time window of a group of measurement points of the peripheral blood pressure curve, wherein a denominator (n) for forming the fraction is between 3 and 6, in particular between 3.8 and 4.2, wherein
   the window width (N) is corrected with a pulse-dependent correction factor to yield a pulse-corrected window width (N$_1$).

2. The method according to claim 1, **characterized in that** the peripheral blood pressure curve is determined relatively, a systolic pressure and a diastolic pressure of the patient are determined as absolute values, and an absolute peripheral blood pressure curve is determined by calibrating the relative blood pressure curve with the systolic and diastolic pressure values

3. The method according to claim 1 or 2, **characterized in that** the relative peripheral blood pressure curve, the systolic pressure, the diastolic pressure and/or the pulse of the patient is/are measured using a sphygmomanometer.

4. The method according to at least one of the preceding claims, **characterized in that** the window width (N) is plotted with the width N = 2m + 1 on the time axis about a respective measurement point of the measured peripheral blood pressure curve, where m is an integral variable for determining a number of measurement points.

5. The method according to at least one of the preceding claims, **characterized in that** the all-pass filter is created by adding an offset to the average-filtered blood pressure curve.

6. The method according to claim 4 or 5, **characterized in that** the filtered blood pressure curve (y(k)) is determined according to a first filter equation (equation 1)

$$y(k) = A \cdot x(k) + B \cdot \frac{1}{2m+1} \cdot \sum_{i=-m}^{m} x(k+i)$$

or according to a second filter equation (equation 4)

$$y(k) = A \cdot x(k) + B \cdot \frac{1}{2m+1} \cdot \sum_{i=-m}^{m} x(k + i - \varphi)$$

where A denotes an all-pass coefficient, and B denotes an average filter coefficient, where A + B = 1, x(k) is the absolute peripheral blood pressure curve, k is the index of the measurement points of the peripheral blood pressure curve, and i is the index of neighboring measurement points for determination of the average, and $\varphi$ is a phase angle.

7. The method according to claim 6, **characterized in that** the all-pass coefficient (A), the average filter coefficient (B), the window width (N) and/or the denominator (n) are determined by approximating a transfer function (H(f)), which corresponds to the filter equation (equation 1), to a calibration transfer function.

8. The method according to claim 7, **characterized in that** a generalized transfer function, determined by a patient base, is used as the calibration transfer function.

9. The method according to at least one of the preceding claims,
**characterized in that** the pulse-corrected window width ($N_1$) is calculated by using a correction (equation 3), according to which it holds that:

$$N = \frac{|f_s|}{nk} \cdot \frac{1}{HR_{Pat}/HR_{Ref}}$$

where $f_S$ is the scanning frequency in Hertz, n is the denominator, k is a correction factor, $HR_{Pat}$ is the patient's instantaneous heart rate, and $HR_{Ref}$ is the reference heart rate.

10. A device for determining the central systolic blood pressure of a patient, comprising a blood pressure measurement device for determining a peripheral blood pressure curve, in particular a brachial blood pressure curve and an evaluation unit configured for

 - applying a moving-average filter to the blood pressure curve,
 - applying an all-pass filter to the blood pressure curve,
 - determining the central systolic blood pressure as the maximum of the filtered blood pressure curve, wherein

the moving-average filter is applied in such a way that a fraction of the value of the scanning frequency ($f_S$) for measuring the peripheral blood pressure curve is selected as a window width (N) of a time window of a group of measurement points of the peripheral blood pressure curve, wherein a denominator (n) for forming the fraction is between 3 and 6, in particular between 3.8 and 4.2, wherein
the window width (N) is corrected using a pulse-dependent correction factor to yield a pulse-corrected window width ($N_1$).

11. The device according to claim 10, **characterized in that** the blood pressure measuring device is suitable for determining the peripheral blood pressure curve as a relative value and for determining the patient's diastolic pressure as an absolute value, and the evaluation unit is configured for determining an absolute peripheral blood pressure curve by calibration of the relative blood pressure curve with the absolute systolic pressure and diastolic pressure.

12. The device according to claim 10 or 11, **characterized in that** the evaluation unit is configured to carry out a method according to at least one of claims 1 through 9.

13. A blood treatment device according to at least one of claims 10 through 12, having an extracorporeal blood circulation, for treatment of a patient's blood, and having a device for determining the patient's central systolic blood pressure.

**Revendications**

1. Procédé de détermination de la tension artérielle systolique centrale d'un patient, présentant les étapes de

 - détermination d'une évolution périphérique de tension artérielle, en particulier brachiale,

- application d'un filtre glissant de valeur moyenne sur l'évolution de la tension artérielle,
- application d'un filtre passe-tout sur l'évolution de la tension artérielle,
- détermination de la tension artérielle systolique centrale sous forme de maximum de l'évolution de tension artérielle filtrée par filtre passe-tout et valeur moyenne, le filtre glissant de valeur moyenne étant appliqué de manière à ce qu'une fraction de la valeur d'une fréquence de balayage ($f_s$) pour mesurer l'évolution de tension artérielle périphérique sous forme d'une largeur de fenêtre (N) d'une fenêtre temporelle d'un groupe de points de mesure de l'évolution de tension artérielle périphérique soit sélectionnée, un dénominateur (n) de calcul de la fraction étant situé entre 3 et 6, en particulier entre 3,8 et 4,2,

la largeur de fenêtre (N) étant corrigée avec un facteur de correction dépendant du pouls pour obtenir une largeur de fenêtre à pouls corrigé ($N_1$).

2. Procédé selon la revendication 1, **caractérisé en ce que** l'évolution périphérique de tension artérielle est déterminée de manière relative, une tension systolique et diastolique du patient sont déterminées de manière absolue, et une évolution périphérique absolue de tension artérielle est déterminée par étalonnage de l'évolution relative de tension artérielle avec la tension systolique et diastolique.

3. Procédé selon la revendication 1 ou 2, **caractérisé en ce que** l'évolution relative périphérique de tension artérielle, la tension systolique, la tension diastolique et/ou le pouls du patient sont mesurés au moyen d'un sphygmomanomètre.

4. Procédé selon au moins une des revendications précédentes, **caractérisé en ce que** la largeur de fenêtre (N) est établie avec la largeur N = 2m + 1 sur l'axe temporel autour d'un point de mesure respectif de l'évolution périphérique mesurée de tension artérielle, m étant une variable en nombre entier pour la fixation d'un certain nombre de points de mesure.

5. Procédé selon au moins une des revendications précédentes, **caractérisé en ce que** le filtre passe-tout est appliqué en ajoutant un décalage sur l'évolution de tension artérielle filtrée par valeur moyenne.

6. Procédé selon la revendication 4 ou 5, **caractérisé en ce que** l'évolution de tension artérielle filtrée (y(k)) est déterminée d'après une première équation de filtrage (éq. 1)

$$y(k) = A \cdot x(k) + B \cdot \frac{1}{2m+1} \cdot \sum_{i=-m}^{m} x(k+i)$$

ou d'après une seconde équation de filtrage (éq. 4)

$$y(k) = A \cdot x(k) + B \cdot \frac{1}{2m+1} \cdot \sum_{i=-m}^{m} x(k+i-\varphi),$$

sachant que A est un coefficient de passe tout et B un coefficient de filtrage de valeur moyenne, sachant que A + B = 1, x(k) est l'évolution périphérique absolue de tension artérielle, k l'index des points de mesure de l'évolution périphérique de tension artérielle es i l'index de points de mesure voisins pour la détermination de la valeur moyenne et $\varphi$ un angle de phase.

7. Procédé selon la revendication 6, **caractérisé en ce que** le coefficient passe-tout (A), le coefficient de filtrage de valeur moyenne (B), la largeur de fenêtre (N) et/ou le dénominateur (n) sont déterminés en rapprochant une fonction de transfert (H(f)) correspondant à l'équation de filtrage (éq. 1) d'une fonction de transfert d'étalonnage.

8. Procédé selon la revendication 7, **caractérisé en ce qu'**on utilise comme fonction de transfert d'étalonnage une fonction de transfert généralisée définie par un tronc de patient.

9. Procédé selon au moins une des revendications précédentes, **caractérisé en ce que** la largeur de fenêtre à pouls corrigé ($N_1$) est calculée par une équation de correction (éq. 3), selon laquelle :

$$N = \frac{|f_s|}{nk} \cdot \frac{1}{HR_{Pat}/HR_{Ref}}$$

$f_S$ étant la fréquence de balayage en Hertz, n le dénominateur, k un facteur de correction, $HR_{Pat}$ la fréquence cardiaque actuelle du patient et $HR_{Ref}$ la fréquence cardiaque de référence.

10. Dispositif de détermination de la tension artérielle systolique centrale d'un patient, présentant un dispositif de mesure de tension artérielle pour la détermination d'une évolution de tension artérielle périphérique, en particulier brachiale, st une unité d'exploitation qui est configurée pour la

   - application d'un filtre glissant de valeur moyenne sur l'évolution de la tension artérielle,
   - application d'un filtre passe-tout sur l'évolution de tension artérielle,
   - détermination de la tension artérielle systolique centrale sous forme de maximum de l'évolution de tension artérielle, le filtre glissant de valeur moyenne étant appliqué de manière à ce qu'une fraction de la valeur d'une fréquence de balayage (fs) pour mesurer l'évolution de tension artérielle périphérique sous forme d'une largeur de fenêtre (N) d'une fenêtre temporelle d'un groupe de points de mesure de l'évolution

   de tension artérielle périphérique soit sélectionnée, un dénominateur (n) de calcul de la fraction étant situé entre 3 et 6, en particulier entre 3,8 et 4,2, la largeur de fenêtre (N) étant corrigée avec un facteur de correction dépendant du pouls pour obtenir une largeur de fenêtre à pouls corrigé ($N_1$).

11. Dispositif selon la revendication 10, **caractérisé en ce que** le dispositif de mesure de tension artérielle est apte à déterminer l'évolution périphérique de tension artérielle de manière relative et à déterminer de manière absolue une tension systolique et une tension diastolique du patient, et que l'unité d'exploitation est configurée pour déterminer une évolution périphérique absolue de la tension artérielle par étalonnage de l'évolution relative de tension artérielle avec la tension systolique et diastolique absolue.

12. Dispositif selon la revendication 10 ou 11, **caractérisé en ce que** l'unité d'exploitation est configurée pour réaliser un procédé selon au moins une des revendications 1 à 9.

13. Dispositif de traitement sanguin comportant un circuit sanguin extracorporel, pour le traitement du sang d'un patient, présentant un dispositif de détermination de la tension artérielle systolique centrale du patient selon au moins une des revendications 10 à 12.

**Fig. 1**

**Fig. 2**

Gespeicherte verfahrensrelevante Parameter:

- Konstante A des Allpassfilters
- Konstante B des gleitenden Mittelwertfilters
- Referenzherzrate 70 bpm ($HR_{70}$) und Kernbereich der Herzrate von [$HR_L$, $HR_H$]
- Fensterbreite N des Filters bei $HR_{70}$

↓

oszillometrische Blutdruckmessung; daraus Bestimmung des systolischen ($SBP_0$) und diastolischen Blutdrucks ($DBP_0$) und der Herzrate ($HR_{Pat}$)

↓

Aufpumpen der Manschette auf den Druck vom $DBP_0$ und Halten des Drucks für ca. 10 Sekunden

↓

Erfassung der Druckpulsationen (des Drucksignals) während der Druckhaltung

↓

Eliminierung des Gleichanteils und der Hochfrequenz-störungen (f > 18 Hz) des Drucksignals mit Hilfe digitaler Filter zur Gewinnung der peripheren Pulskurven

↓

Bildung einer gemittelten Pulskurve nach Bewertung und Selektierung der peripheren Pulskurven

↓

Kalibrierung der gemittelten Pulskurve mit $SBP_0$ und $DBP_0$ zur Gewinnung der peripheren Blutdruckkurve

↓

Anpassung der Fensterbreite N an Herzrate, wenn aktuelle Herzrate ($HR_{Pat}$) außerhalb des Kernbereichs [$HR_L$, $HR_H$]

↓

Applikation des Allpass- und des gleitenden Mittelwertfil-ters an die periphere Blutdruckkurve zur Gewinnung der zentralen Blutdruckkurve

↓

Bestimmung des Maximums der zentralen Blutdruckkurve als der zentrale systolische Blutdruck

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KARAMANOGLU et al.** An analysis of the relationship between central aortic and peripheral upper limb pressure waves in man. *European Heart Journal,* 1993, vol. 14, 160-167
- **Y.-T. SHIH et al.** Application of the N-Point Moving Average Method for Brachial Pressure Waveform-Derived Estimation of Central Aortic Systolic Pressure. *HYPERTENSION,* 01. April 2014, vol. 63 (4), 865-870
- **Y.-T. SHIH et al.** Comparison of two generalized transfer functions for measuring central systolic blood pressure by an oscillometric blood pressure monitor. *Journal of Human Hypertension,* 2013, vol. 3, 204-210